# EUROPEAN PATENT APPLICATION

(11) **EP 2 923 656 A1**
(43) Date of publication of application: **30.09.2015**
(21) Application number: 13856141.0
(22) Date of filing: 18.11.2013
(51) Int. Cl.: A61B 17/072, A61B 17/12, A61B 17/3211, A61B 18/12

(54) **TISSUE ABLATION APPARATUS**

(30) Priority: 20.11.2012 US 201261728507 P
(71) Applicant: OLYMPUS CORPORATION, Shibuya-ku Tokyo 151-0072 (JP)
(72) Inventor: MIYAMOTO Manabu, Tokyo 151-0072 (JP); BANJU Kazuo, Tokyo 151-0072 (JP); TAKEMOTO Shotaro, Tokyo 151-0072 (JP); TAKAHASHI Shinji, Tokyo 151-0072 (JP); MIKKAICHI Takayasu, Tokyo 151-0072 (JP); SUZUKI Satoko, Tokyo 151-0072 (JP)
(74) Representative: Schicker, Silvia
(86) International application number: PCT/JP2013/081020
(87) International publication number: WO 2014/080862

(57) **Abstract**

A tissue ablation apparatus includes a pair of grasping members that holds a tissue; and a cartridge that is replaceably attached to the pair of grasping members. The cartridge includes a cartridge body that has a longitudinal axis and a slot extending along the longitudinal axis, guides a cutting member cutting a tissue by the slot, and defines a movement range of the cutting member; a pair of sealing portions that are provided on both sides of the slot so as to sandwich the slot and extend parallel to the slot, respectively; and a tissue sealing portion that is located closer to a distal end side than the slot and is provided on an extension line of the slot.

## Description

### Technical Field

The present invention relates to a tissue ablation apparatus, and more particularly, a tissue ablation apparatus to be used for all-layer ablation that ablates a portion of a lumen tissue over an entire thickness direction.

Priority is claimed on United States Patent Application No. 61/728,507, provisionally applied in United States on November 20, 2012, the content of which is incorporated herein by reference.

### Background Art

In the related art, in the medical treatment of stomach cancer or the like, ablating a tumor and its surrounding tissue over an entire thickness direction of the stomach wall is performed. Such all-layer ablation is often performed by a laparotomy or laparoscopic surgery.

Additionally, a stapler for operation equipped with a pair of jaws is described in Patent Document 1. One of the pair of jaws is mounted with a cartridge loaded with staples, and an anvil member that has a plurality of staple pockets is attached to the other jaw. If the jaws are closed with a tissue interposed between the pair of jaws, a portion of the tissue can be ablated over all the layers by a built-in cutter while suturing the tissue with staples.

### Citation List

### Patent Literature

[Patent Document 1] Published Japanese Translation No. 2010-522035 of the PCT International Publication

### Summary of the Invention

### Technical Problem

The related-art all-layer ablation has been performed through a laparotomy or laparoscopic surgery. However, it is studied that this ablation is performed through natural openings, such as a mouth, from viewpoints of making invasion to a patient smaller or of reducing the risk of recurrence when a tumor is ablated.

However, in the related-art tissue ablation apparatus as described in Patent Document 1, only first staple rows are provided. Therefore, if suturing and cutting are performed along first pull-in lines, a tissue between the first staple rows on a distal end side of (ahead of) the cutting line of the cutting member is not fastened, and a region that becomes a hole-like state remains. Therefore, for example, when the internal pressure of the lumen tissue becomes high, there is a problem in that a state where there is a concern that the contents of the lumen tissue may leak out to the outside from the relevant region may occur, though the leak-out is temporary.

### Solution to Problem

A tissue ablation apparatus of a first aspect of the present invention includes a pair of grasping members that holds a tissue; and a cartridge that is replaceably attached to the pair of grasping members. The cartridge includes a cartridge body that has a longitudinal axis and a slot extending along the longitudinal axis, guides a cutting member cutting a tissue by the slot, and defines a movement range of the cutting member; a pair of sealing portions that are provided on both sides of the slot so as to sandwich the slot and extend parallel to the slot; and a tissue sealing portion that is provided closer to a distal end side than the slot and is provided on an extension line of the slot.

According to the tissue ablation apparatus of a second aspect, in the first aspect, the tissue sealing portion may be held between the pair of grasping members, and may join the tissue located closer to the distal end side than the slot.

According to the tissue ablation apparatus of a third aspect, in the first aspect, the pair of sealing portions may extend toward the distal end side than the slot, and the tissue sealing portion may be provided between the pair of sealing portions.

According to the tissue ablation apparatus of a fourth aspect, in the first aspect, the pair of sealing portions may be a pair of staple rows configured by a plurality of staples arranged within the cartridge body.

According to the tissue ablation apparatus of a fifth aspect, in the first aspect, the tissue sealing portion may be a sealing staple arranged on the distal end side of the slot within the cartridge body.

According to the tissue ablation apparatus of a sixth aspect, in the first aspect, the tissue sealing portion may be a pair of electrodes that are held between the pair of grasping members and are capable of coming into contact with the tissue located closer to the distal end side than the slot.

According to the tissue ablation apparatus of a seventh aspect, in the first aspect, the tissue sealing portion may be a pair of heat generating elements that are held between the pair of grasping members and are capable of coming into contact with the tissue located closer to the distal end side than the slot.

A tissue ablation apparatus of an eighth aspect of the present invention includes an insertion section that extends along a longitudinal axis; a pair of grasping members that are provided at a distal end of the insertion section and grasp a tissue; a cutting member that moves along a predetermined flow line to thereby cut a lumen tissue held between the pair of jaws; a pair of sealing portions that are provided on both sides of the flow line so as to sandwich the flow line and extend parallel to the flow line; and a tissue sealing portion that is provided on an extension line of the flow line ahead of the flow line.

According to the tissue ablation apparatus of a ninth aspect, in the eighth aspect, the tissue sealing portion may be held between the pair of grasping members, and may join the tissue located closer to the distal end side than the flow line.

According to the tissue ablation apparatus of a tenth aspect, in the eighth aspect, the pair of sealing portions may extend toward the distal end side than the flow line, and the tissue sealing portion may be provided between the pair of sealing portions.

According to the tissue ablation apparatus of an eleventh aspect, in the eighth aspect, the pair of sealing portions may be a pair of staple rows configured by a plurality of staples arranged on both sides of the flow line.

According to the tissue ablation apparatus of a twelfth aspect, in the eighth aspect, the tissue sealing portion may be a sealing staple arranged on the distal end side of the flow line.

According to the tissue ablation apparatus of a thirteenth aspect, in the eighth aspect, the tissue sealing portion may be a pair of electrodes that are held between the pair of grasping members and are capable of coming into contact with the tissue located closer to the distal end side than the flow line.

According to the tissue ablation apparatus of a fourteenth aspect, in the eighth aspect, the tissue sealing portion may be a pair of heat generating elements that are held between the pair of grasping members and are capable of coming into contact with the tissue located closer to the distal end side than the flow line.

### Advantageous Effects of the Invention

In the tissue ablation apparatuses of the above respective aspects, since the tissue sealing portion is arranged in the cartridge, a tissue between the pair of sealing portions on the distal end side of (ahead of) the cutting line of the cutting member is reliably fastened and sealed by the tissue sealing portion. Additionally, the holding using the pair of grasping members is released after a tissue is first fastened by the pair of sealing portions and the tissue sealing portion and the cutting member cuts the tissue, in a state where the tissue is pulled into a narrow gap between the pair of grasping members and grasped by the grasping members. Accordingly, tissue ablation can be performed in a series of procedures using the tissue ablation apparatus without completely causing a state where there is a concern that the contents of the lumen tissue may leak out to the outside.

### Brief Description of Drawings

FIG. 1 is a view illustrating an overall configuration of a tissue ablation apparatus of a first embodiment of the present invention.
FIG. 2 is a view illustrating one operation when the tissue ablation apparatus is used.
FIG. 3 is a partially enlarged view illustrating a state where a first jaw and a cartridge of the tissue ablation apparatus are viewed from a second jaw side.
FIG. 4A is a view illustrating a mechanism in which staples are shot from the cartridge.
FIG. 4B is a view illustrating drivers within the cartridge.
FIG. 5 is a view illustrating an ablation region and a grasped point.
FIG. 6 is a view illustrating one operation when the tissue ablation apparatus is used.
FIG. 7 is a view illustrating one operation when the tissue ablation apparatus is used.
FIG. 8 is a view illustrating one operation when the tissue ablation apparatus is used.
FIG. 9 is a view illustrating one operation when the tissue ablation apparatus is used.
FIG. 10 is a view illustrating one operation when the tissue ablation apparatus is used.
FIG. 11 is a view illustrating one operation when the tissue ablation apparatus is used.
FIG. 12 is a view illustrating a state after suturing and cutting is performed using a related-art tissue ablation apparatus.
FIG. 13 is a view illustrating a cutter in a modification example of the embodiment.
FIG. 14 is a partially enlarged view illustrating a distal end side of the first jaw and a cartridge in the modification example of the embodiment.
FIG. 15 is a partially enlarged view illustrating a distal end side of a first jaw and a cartridge in a tissue ablation apparatus of a second embodiment of the present invention.
FIG. 16 is a view illustrating electrical connection in the tissue ablation apparatus.
FIG. 17 is a partially enlarged view illustrating a distal end side of a first jaw and a cartridge in a modification example of the embodiment.

### Description of Embodiments

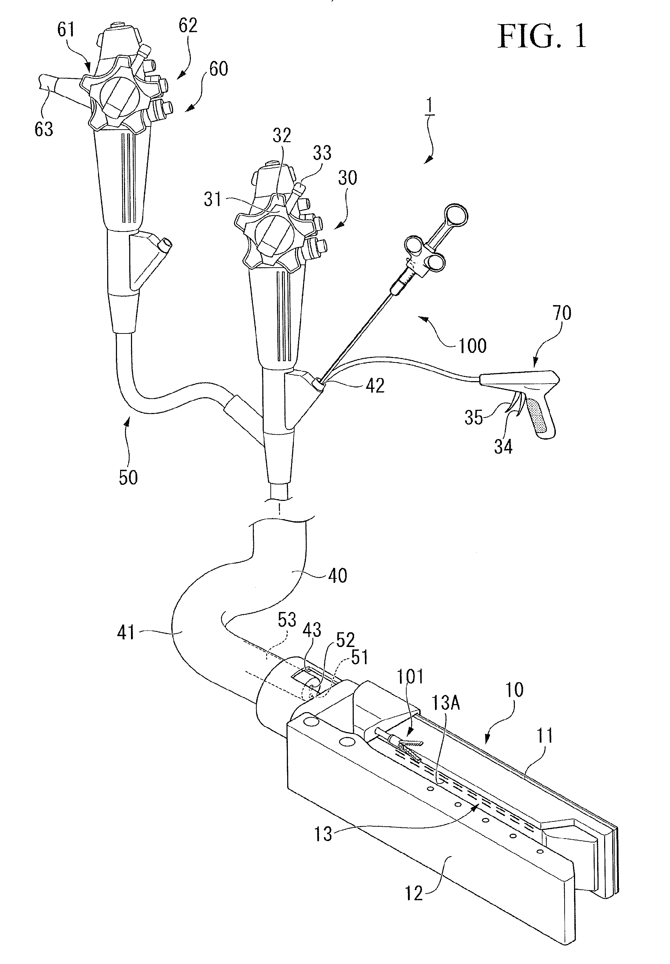

Hereinafter, a first embodiment of the present invention will be described with reference to FIGS. 1 to 14.

FIG. 1 is a view illustrating an overall configuration of a tissue ablation apparatus 1 of the present embodiment. The tissue ablation apparatus 1 includes a treatment section 10 that is provided at a distal end to perform a treatment on a target tissue. Additionally, the tissue ablation apparatus 1 includes a first operation section 30 for operating the treatment section 10, an elongated insertion section 40 provided between the treatment section 10 and the first operation section 30, an observation section 50 inserted through the insertion section 40, a second operation section 60 for operating the observation section 50, and a third operation section 70 for operating the treatment section. The treatment section 10 has a first jaw (first grasping member) 11 and a second jaw (second grasping member) 12 as a pair of openable and closable jaws, and sutures and detaches a tissue, using a cartridge 13 loaded with staples 13A. Additionally, the basic structure of the treatment section 10 is well-known, for example, as described in Patent Document 1.

The first operation section 30 has a well-known configuration and has two dial knobs 31 and 32 and locking levers 33. The dial knobs 31 and 32 are connected to a bending portion 41 (to be described below) by an operating member (not shown), such as a wire. The third operation section 70 is provided with a first trigger 34 for operating the opening and closing of the pair of jaws 11 and 12 and a second trigger 35 for performing suture and incision operations.

The insertion section 40 has flexibility and is formed in a tubular shape that extends along a longitudinal axis. The treatment section 10 is attached to a distal end side of the insertion section 40, and the first operation section 30 is attached to a proximal end side of the insertion section 40. The insertion section 40 has the bending portion 41 of a well-known structure having a plurality of joint rings, bending pieces, or the like on the distal end side thereof, and can be bent by operating the dial knobs 31 and 32 of the first operation section 30. The bent state can be fixed by operating the locking lever 33. The operating member is inserted through an inner cavity of the insertion section 40 so as to be able to advance and retract in an axial direction. Additionally, a forceps port 42 is provided on the proximal end side of the insertion section 40, and a general treatment tool 100 or the like for an endoscope equipped with a forceps portion 101 can be inserted into the forceps port 42 and can be protruded from the proximal end side of the first jaw 11.

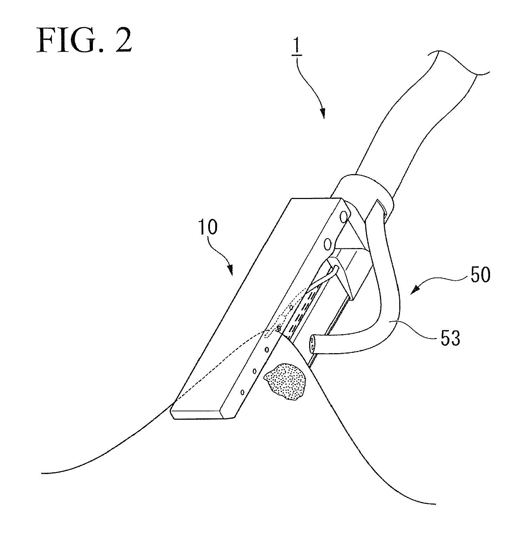

The observation section 50 is inserted through the insertion section 40 so as to be able to advance and retract, and includes an illumination unit 51 including an LED or the like and imaging means 52, such as a CCD, at a distal end portion thereof. Additionally, the observation section has a bending portion 53 having the same structure as the bending portion 41 on the distal end side thereof. A distal end portion of the observation section 50 can be protruded and retracted from an opening 43 provided on the distal end side of the insertion section 40. Accordingly, for example, as shown in FIG. 2, a tissue held by the treatment section 10 can be favorably observed by protruding the distal end portion of the observation section 50 and appropriately bending the bending portion 53.

The second operation section 60 is connected to a proximal end of the observation section 50 that comes out of the proximal end side of the insertion section 40. The second operation section 60 is provided with the same dial knob 61, button 62, or the like as those of the first operation section 30. The second operation section 60 can perform the bending operation of the bending portion 53, the operation of the illumination unit 51 and the imaging means 52, or the like. A video signal acquired by the imaging means 52 is sent to an image processor (not shown) through a universal cable 63, and is displayed on a display (not shown) or the like. As the observation section 50 and the second operation section 60, a well-known endoscope apparatus or the like can be used by appropriately setting dimensions or the like.

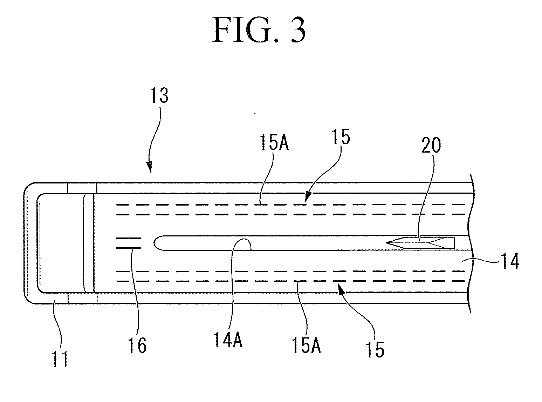

FIG. 3 is a partially enlarged view illustrating a state where the first jaw 11 and the cartridge 13 are viewed from the second jaw 12 side. The cartridge 13 includes a cartridge body 14 having a longitudinal axis and a slot 14A extending along the longitudinal axis for allowing a cutter (cutting member) 20 that cuts a tissue to move therealong, a pair of first staple rows (a pair of sealing portions) 15 that extends parallel to the slot 14A on both sides of the slot 14A in a width direction, respectively, and second staples (tissue sealing portion, sealing staples) 16 arranged on an extension line of the slot 14A ahead of (closer to the distal end side than the slot 14A) of the slot 14A.

A plurality of staples 15A are aligned and arranged within the cartridge body 14 in the first staple row 15, and extend further toward the distal end side than the slot 14A by a predetermined length, for example, 5 millimeters (mm). The second staples 16 are arranged between the two first staple rows 15. Although FIG. 3 shows an example in which two second staples 16 are arranged, the number of second staples to be arranged is not particularly limited, and may be one or may be three or more. The staples to be arranged as the second staples may be the same staples 15A as the first staple rows, and may be different therefrom.

The cutter 20 is inserted through the slot 14A from the inside of the cartridge 13, and moves within the slot 14A in a state where the cutter has protruded toward the second jaw 12. The cutter 20 moves along the slot 14A in a state where a protruding end of the cutter 20 has entered a groove provided in an anvil member to be described below so that a tissue sandwiched between the pair of jaws 11 and 12 can be cut over all layers. The blade length of the cutter 20 required for this is greater than a thickness equivalent to two sheets of target tissues held in a folded state and is greater than the distance between the pair of jaws 11 and 12 in a closed state.

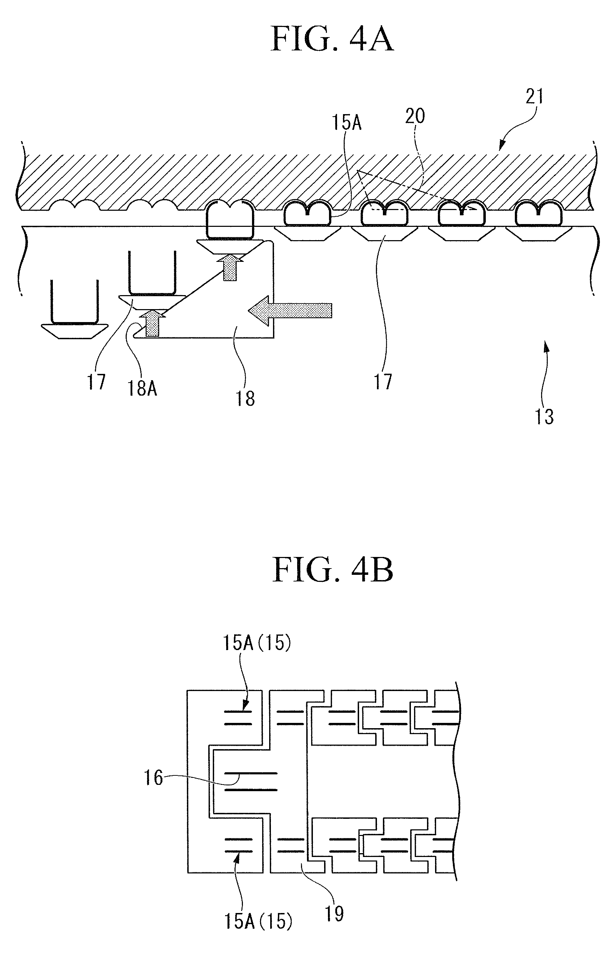

FIG. 4A is a view illustrating a mechanism in which staples are shot from the cartridge 13. Drivers 17 formed of resin or the like are arranged under the staples 15A. A wedge 18 having an inclined surface 18A on a distal end side is arranged within the cartridge 13. If the second trigger 35 is operated to advance the wedge 18, the wedge 18 pushes up drivers 17 that have come into contact with the inclined surface 18A. As a result, the staples 15A are shot sequentially from the proximal end side by being pushed by the drivers 17 and pass through a tissue, and end portions of the staples passed through the tissue are pressed against and folded by the anvil member 21 provided at the second jaw 12.

Although the drivers 17 are respectively arranged at the two first staple rows 15, as shown in FIG. 4B, a driver 19 that pushes up the two first staple rows 15 and the second staples 16 are arranged in a region closer to the distal end side than the slot 14A. Accordingly, if the wedge 18 pushes up the driver 19, the staples 15A of the first staple rows 15 and the second staples 16 are pushed up and shot together.

Next, the operation when the tissue ablation apparatus 1 is used will be described taking a case where all the layers of a malignant tumor (hereinafter simply referred to as a "tumor") of the stomach (lumen tissue) are ablated as an example.

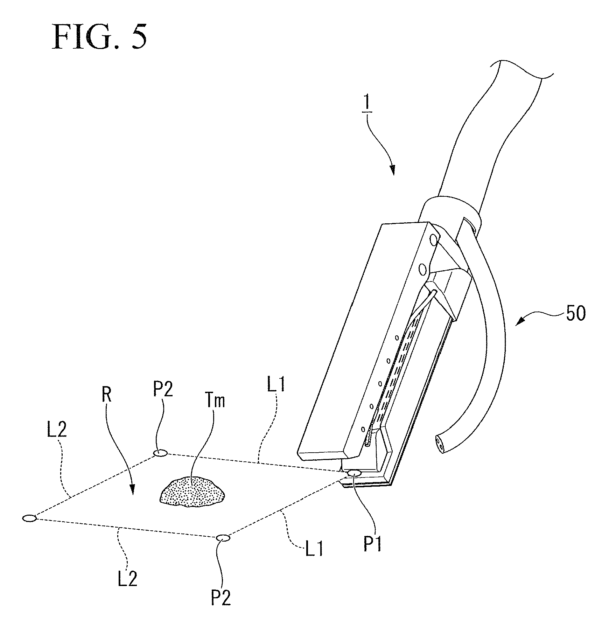

First, a surgeon introduces the tissue ablation apparatus 1 into the stomach from a patient's mouth or the like, and observes the tumor, using the observation section 50. Then, as shown in FIG. 5, a rough ablation region R surrounding a tumor Tm is set. It is preferable that the ablation region R be set so that the shortest distance from the tumor Tm becomes equal to or greater than 5 millimeters (mm) so as not to damage the tumor Tm at the time of tissue ablation.

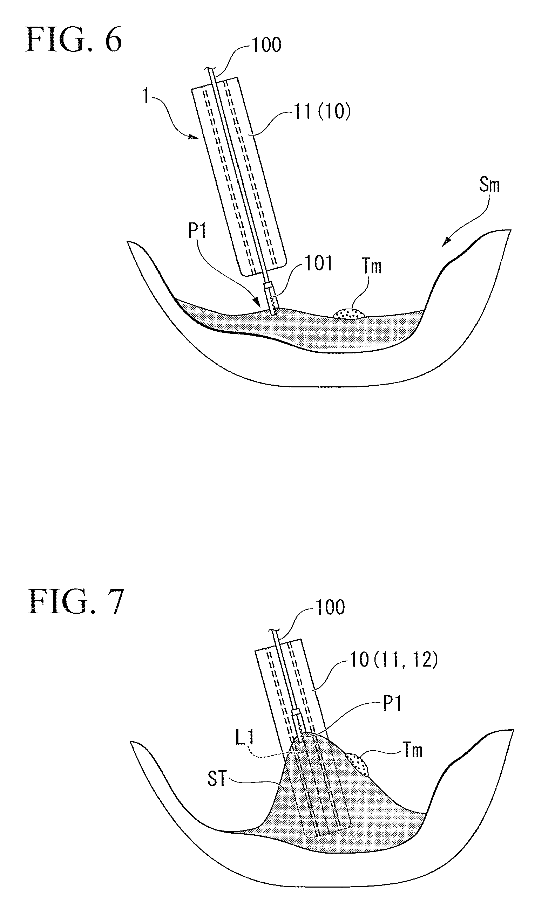

Next, the surgeon protrudes the treatment tool 100 for an endoscope from the first jaw 11, and as shown in FIG. 6, grasps one point of a stomach wall apart from the tumor Tm, using the forceps portion 101, from the inside of the stomach Sm. Since this one point becomes a portion of a peripheral edge of a region to actually be ablated, it is preferable that this point be set to a position equivalent to a peripheral edge portion of the set ablation region R, for example, a part apart from the center of the tumor Tm by about 30 mm can be selected. Hereinafter, this one grasped point is referred to as a first grasped point P1.

Subsequently, the surgeon retracts the treatment tool 100 for an endoscope that has grasped the first grasped point P1, and as shown in FIG. 7, pulls a portion of a stomach wall tissue ST into a gap between the first jaw 11 and the second jaw 12 while appropriately operating the first trigger 34. During the pull-in, the tumor Tm is observed by the observation section 50, and attention is paid so that the tumor Tm is not touched by the pair of jaws.

The stomach wall tissue ST is pulled in between the first jaw 11 and the second jaw 12 along two first pull-in lines L1 that extend from the first grasped point P1. That is, the pulled-in stomach wall tissue ST is folded with the external surface of the stomach turned inward so that the external surfaces of the stomach come into contact with each other, and is pulled in in a state where one pull-in line of the first pull-in lines L1 faces the first jaw 11 and the other pull-in line faces the second jaw 12. Since the gap between the pair of jaws is narrow slit-shaped and the spacing therebetween is a thickness equivalent to approximately two sheets of stomach wall tissues, tissues of other internal organs adjacent to the stomach are not pulled in between the pair of jaws together with the stomach wall tissues.

If the stomach wall tissue ST is sufficiently pulled in between the pair of jaws 11 and 12, the surgeon operates the second trigger 35. Accordingly, the wedge 18 advances toward a distal end within the cartridge 13, and the drivers 17 arranged under the respective staples 15A of the first staple rows 15 are pushed up sequentially from the proximal end side. As a result, the staples 15A are pressed against the anvil member 21 (refer to FIG. 4A) sequentially provided at the second jaw 12 from the proximal end side toward the distal end side, and both the end portions of the staples 15A passed through the stomach wall tissue ST are folded. As a result, the folded stomach wall tissue ST is fastened so as to become integral along the thickness direction on both sides of the first pull-in lines L1.

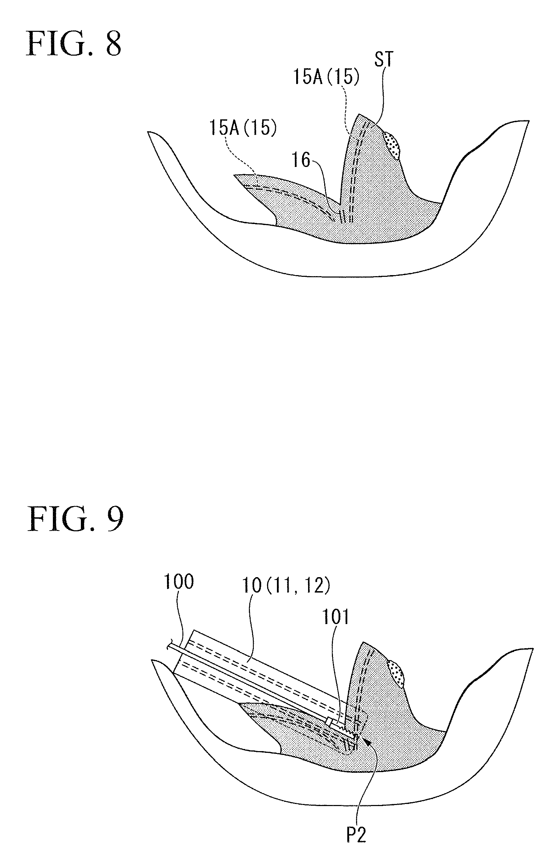

Moreover, the cutter 20 (refer to FIG. 4A) advances toward the distal end slightly later than the wedge 18, and cuts the stomach wall tissue located between the first staple rows 15 that has fastened the stomach wall tissue ST, substantially along the first pull-in lines L1. As a result, as shown in FIG. 8, a portion of the stomach wall is cut over all the layers. Here, the stomach wall tissue located between the first staple rows 15 on the front on an extension line of a flow line (substantially the same as the slot 14A) of the cutter 20 is integrally fastened by the second staples 16.

After the cutting and suturing along the first pull-in lines L1 are completed, the surgeon extracts the tissue ablation apparatus 1 out of the body cavity, replaces the cartridge 13 with a new cartridge loaded with staples, and introduces the new cartridge again into the stomach. Then, as shown in FIG. 9, the stomach wall tissue is grasped by the forceps portion 101 of the treatment tool 100 for an endoscope, with an end portion of a cutting line along the first pull-in lines L1 opposite to the first grasped point P1 as a second grasped point P2.

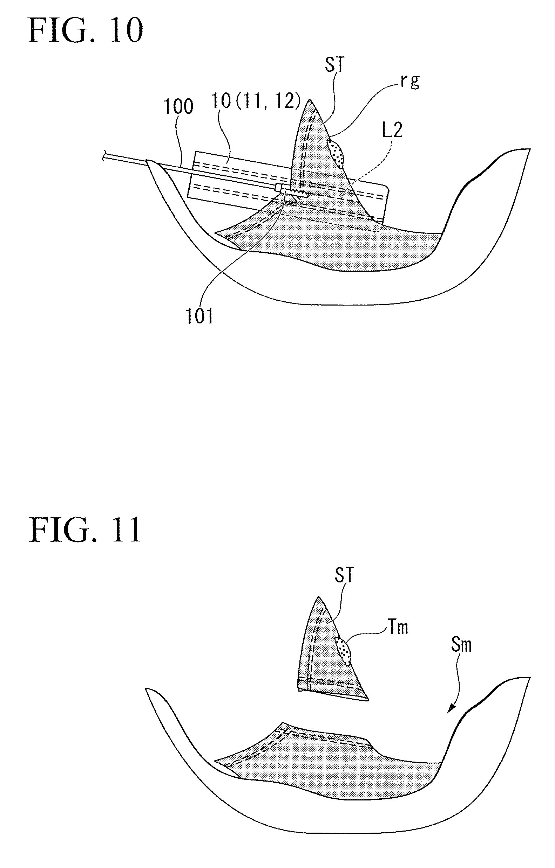

Next, the surgeon retracts the treatment tool 100 for an endoscope, and as shown in FIG. 10, pulls the stomach wall tissue ST grasped by the forceps portion 101 into the pair of jaws 11 and 12 along second pull-in lines L2. In this case, the stomach wall tissue ST is pulled in so that a ridgeline rg of the stomach wall tissue that is folded so that the external surfaces of the stomach come into contact with each other passes through the inside of the pair of jaws 11 and 12.

Thereafter, if suturing and cutting are performed by the same operation as the above-described operation, the stomach wall tissue ST is cut and fastened along the second pull-in lines L2. Moreover, since the first pull-in lines L1 and the second pull-in lines L2 form a closed quadrangle as shown in FIG. 5, the stomach wall tissue ST within the ablation region R including the tumor Tm is cut and separated from the stomach Sm, as shown in FIG. 11, by the cutting line formed along the first pull-in lines L1 and the second pull-in lines L2. In this way, a portion of the stomach wall tissue ST is ablated over all the layers without leaving a hole in the stomach Sm.

After the completion of the ablation, the surgeon extracts the tissue ablation apparatus 1, collects the ablated stomach wall tissue ST (ablated piece), and completes the procedure. In this case, the ablated piece may be grasped by the forceps portion 101, and the extraction of the tissue ablation apparatus 1 and the collection of the ablated piece may be simultaneously performed.

As described at the beginning, the related-art all-layer ablation has been performed through a laparotomy or laparoscopic surgery. However, it is studied that this ablation is performed through natural openings, such as a mouth, from viewpoints of making invasion to a patient smaller or of reducing the risk of recurrence when a tumor is ablated.

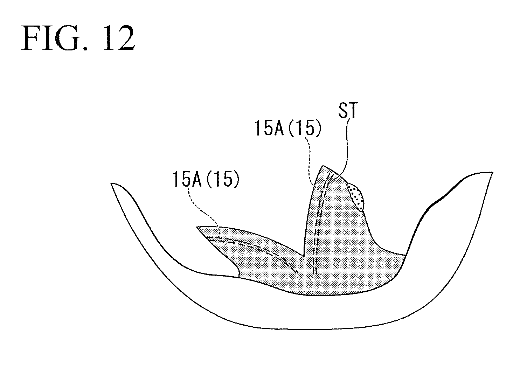

However, in the related-art tissue ablation apparatus described in the above Patent Document 1, only the first staple rows are provided. Therefore, if suturing and cutting are performed along the first pull-in lines, as shown in FIG. 12, a tissue between the first staple rows ahead of the cutting line of the cutter is not fastened, and a region that becomes a hole-like state remains. Therefore, for example, when the internal pressure of the lumen tissue becomes high, there is a problem in that a state where there is a concern that the contents of the lumen tissue may leak out to the outside from the relevant region may occur.

According to the tissue ablation apparatus 1 of the present embodiment, since the second staples 16 serving as the tissue sealing portion are arranged in the cartridge 13, a tissue between the first staple rows 15 ahead of the cutting line of the cutter 20 is reliably fastened and sealed by the second staples 16. Additionally, the holding using the pair of jaws is released after a tissue is first fastened by the first staple rows 15 and the second staples 16 and the cutting member cuts the tissue, in a state where the tissue is pulled into a narrow gap between the pair of jaws 11 and 12 and grasped by the jaws. Accordingly, tissue ablation can be performed in a series of procedures using the tissue ablation apparatus 1 without completely causing a state where there is a concern that the contents of the lumen tissue may leak out to the outside.

Additionally, since suturing and cutting are performed after a tissue is pulled into a relatively narrow slit-shaped gap between the pair of closed jaws 11 and 12, even if other internal organs, tissues, or the like that are adjacent to a lumen tissue, such as the stomach, are likely to be pulled in together, this is prevented at an inlet of the gap. Accordingly, even in an approach through a natural opening where the outside of the lumen tissue cannot be visually recognized, the procedures can be performed without erroneously suturing or cutting these internal organs, tissues, or the like together with the lumen tissue.

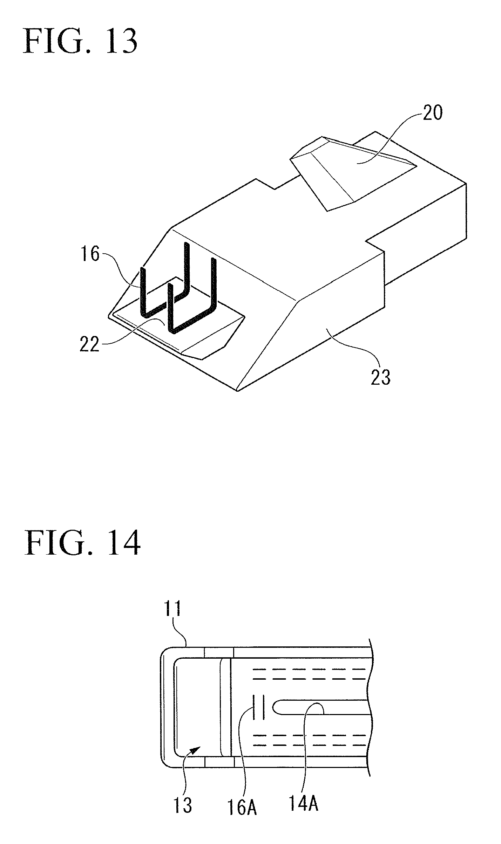

Although an example in which the staples of the first staple rows and the second staples are shot by the common driver closer to the distal end side than the slot 14A has been described in the present embodiment, the structure for shooting the staples is not limited to this. For example, the driver that pushes up the second staples may be separate from the drivers that push up the first staple rows. Specifically, as shown in FIG. 13, a structure may be adopted in which a second wedge 23 is provided ahead of the cutter 20 and a driver 22 that pushes up the second staples 16 is pushed up by the second wedge 23. Otherwise, a configuration may be adopted in which the common driver 19 is pushed up by the second wedge 23, or a structure may be adopted in which all the drivers are pushed up by the second wedge 23.

Additionally, in the above-described example, the orientation of the staples of the first staple rows and the orientation of the second staples are parallel to each other. However, as in a modification example shown in FIG. 14, the longitudinal direction of second staples 16A may be arranged so as to be orthogonal to the slot 14A along which the cutter 20 moves. By adopting such a configuration, tissues ahead of the slot can be joined within a wider range, and tissue ablation can be more reliably performed.

Next, a second embodiment of the present invention will be described. A difference between a tissue ablation apparatus 71 of the present embodiment and the tissue ablation apparatus 1 of the first embodiment is the configuration of a tissue anastomosis portion. In addition, in the following description, components common to those already described will be designated by the same reference numerals, and duplicate description will be omitted.

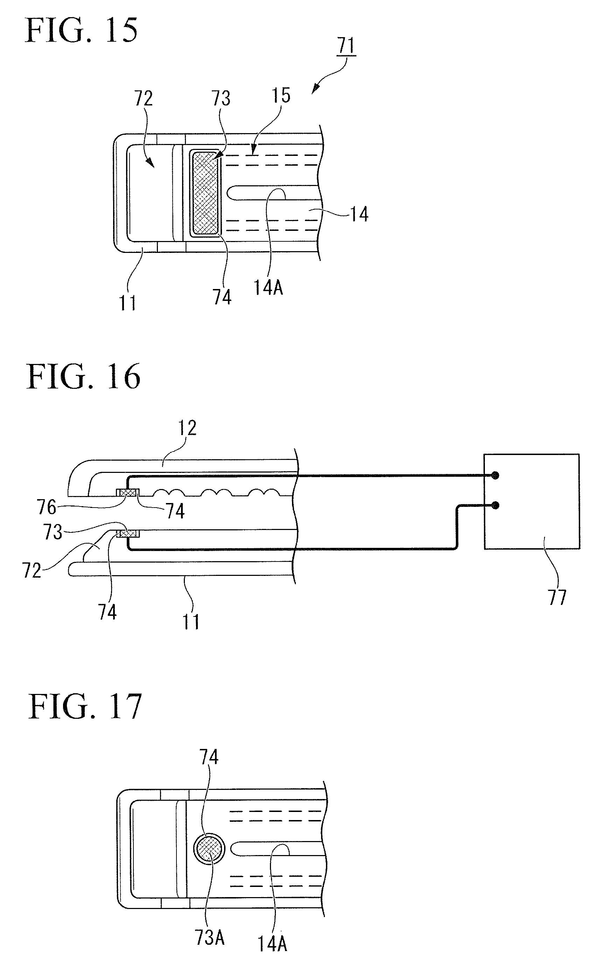

FIG. 15 is a partially enlarged view illustrating the distal end side of the first jaw 11 and a cartridge 72 in the tissue ablation apparatus 71. A linear or beltlike first electrode 73 is provided instead of the second staples ahead of the slot 14A. An insulator 74 is arranged around the first electrode 73 and is insulated from other metal regions in the treatment section 10.

As shown in FIG. 16, a second electrode 76 having the same structure as the first electrode 73 is provided at a position corresponding to the first electrode 73 in an anvil member 75 provided at the second jaw 12, and the first electrode 73 and the second electrode 76 are connected to a high-frequency power source 77. ON/OFF of energization can be switched by an operation section (not shown).

When the tissue ablation apparatus 71 is used, similar to the first embodiment, a tissue is fastened and cut using the first staple rows 15 and the first cutter 20, and then, a high-frequency current is applied to the tissue sandwiched between the first electrode 73 and the second electrode 76 from the high-frequency power source 77 without opening the pair of jaws 11 and 12. Accordingly, the pair of electrodes including the first electrode 73 and the second electrode 76 function as a so-called bipolar electrosurgical instrument. As a result, as described in Japanese Unexamined Patent Application, First Publication No. 2009-247893 or the like, intracellular components and extracelluar components of a tissue that is energized by coming into contact with the first electrode 73 and the second electrode 76 are homogenized (liquidized), tissues are joined together between the pair of jaws 11 and 12, and a hole formed due to the remaining of a non-fastened tissue is sealed.

Even in the tissue ablation apparatus 71 of the present embodiment, similar to the first embodiment, tissue ablation can be performed without completely causing a state where there is a concern that the contents of the lumen tissue may leak out to the outside.

Additionally, since a tissue sealing portion is constituted by the first electrode 73 and the second electrode 76, sealed regions are joined together in a planar fashion. As a result, the hole can be more reliably blocked.

In the present embodiment, the configuration of the tissue sealing portion that seals a contacting tissue through energization is not limited to the above-described bipolar mechanism. For example, a configuration may be adopted in which heat generating elements, such as heaters, which generate heat by energization, are attached to the cartridge and the anvil member instead of the first electrode 73 and the second electrode 76 and a tissue sandwiched between the pair of jaws is heated from both sides. Since these heat generating elements function as a so-called thermal coagulation treatment tool by virtue of this configuration, tissues can be joined together by appropriately setting temperature and heating time.

Additionally, the shape of the electrodes is not limited to the above-described linear shape, belt shape, or the like, and may be a spot shape as in an electrode 73A as shown in FIG. 17.

While the respective embodiments of the present invention have been described above, the technical scope of the present invention is not limited to the above embodiments. Combinations of constituent elements can be changed, various alternations can be added to the respective constituent elements, or omissions can be made, without departing from the concept of the present invention.

For example, although an example in which suturing and cutting are performed twice and a tissue is ablated has been described in the above embodiments, the number of times of suturing and cutting is not limited to twice. For example, in a case where a large ablation region is set due to a large tumor or the like, the ablation region may not be cut in two times depending on the size of the jaws. In such a case, if second suturing and cutting are performed in a state where a portion of a lumen tissue is pulled in so that the ridgeline of the folded lumen tissue does not pass through the inside of the slit, the tissue to be ablated is not cut and separated even after the second suturing and cutting. Thereafter, all layers of a region with an arbitrary size can be ablated by repeating the same suturing and cutting if necessary and finally by performing suturing and cutting such that the ridgeline of the lumen tissue passes through the inside of the slit. Here, since a tissue is not present ahead of the slot in the final suturing and cutting to ablate a tissue piece, the related-art cartridge that does not have the tissue sealing portion may be used.

In addition, the lumen tissue serving as a target of the tissue ablation apparatus of the present invention is not limited to the stomach. For example, although the diameter of the intestines is small compared to that of the stomach, the tissue ablation method of the present invention can be favorably performed even in the intestines by appropriately setting the size of the devices to be used, such as a stapler.

Additionally, it is natural that the diseases of which tissues are to be ablated are also not limited to the tumor described in the embodiments, and for example, can be applied to other diseases, such as a serious ulcer.

### Industrial Applicability

According to the above respective embodiments, since the tissue sealing portion is arranged in the cartridge, a tissue between the pair of sealing portions ahead of the cutting line of the cutting member is reliably fastened and sealed by the tissue sealing portion. Additionally, the holding using the pair of grasping members is released after a tissue is first fastened by the pair of sealing portions and the tissue sealing portion and the cutting member cuts the tissue, in a state where the tissue is pulled into a narrow gap between the pair of grasping members and grasped by the grasping members. Accordingly, tissue ablation can be performed in a series of procedures using the tissue ablation apparatus without completely causing a state where there is a concern that the contents of the lumen tissue may leak out to the outside.

### Reference Signs List

1,71: TISSUE ABLATION APPARATUS
11: FIRST JAW (FIRST GRASPING MEMBER)
12: SECOND JAW (SECOND GRASPING MEMBER) 14: CARTRIDGE BODY
14A: SLOT
15: PAIR OF FIRST STAPLE ROWS (PAIR OF SEALING PORTIONS)
16: SECOND STAPLE (TISSUE SEALING PORTION, SEALING STAPLE)
20: CUTTER (CUTTING MEMBER)

## Claims

1. A tissue ablation apparatus comprising:
a pair of grasping members which holds a tissue; and
a cartridge which is replaceably attached to the pair of grasping members,
wherein the cartridge includes:
a cartridge body that has a longitudinal axis and a slot extending along the longitudinal axis, guides a cutting member cutting a tissue by the slot, and defines a movement range of the cutting member;
a pair of sealing portions that are provided on both sides of the slot so as to sandwich the slot and extend parallel to the slot; and
a tissue sealing portion that is provided closer to a distal end side than the slot and is provided on an extension line of the slot.

2. The tissue ablation apparatus according to Claim 1,
wherein the tissue sealing portion is held between the pair of grasping members, and joins the tissue located closer to the distal end side than the slot.

3. The tissue ablation apparatus according to Claim 1,
wherein the pair of sealing portions extend toward the distal end side than the slot, and
wherein the tissue sealing portion is provided between the pair of sealing portions.

4. The tissue ablation apparatus according to Claim 1,
wherein the pair of sealing portions are a pair of staple rows configured by a plurality of staples arranged within the cartridge body.

5. The tissue ablation apparatus according to Claim 1,
wherein the tissue sealing portion is a sealing staple arranged on the distal end side of the slot within the cartridge body.

6. The tissue ablation apparatus according to Claim 1,
wherein the tissue sealing portion is a pair of electrodes that are held between the pair of grasping members and are capable of coming into contact with the tissue located closer to the distal end side than the slot.

7. The tissue ablation apparatus according to Claim 1,
wherein the tissue sealing portion is a pair of heat generating elements that are held between the pair of grasping members and are capable of coming into contact with the tissue located closer to the distal end side than the slot.

8. A tissue ablation apparatus comprising:
an insertion section that extends along a longitudinal axis;
a pair of grasping members that are provided at a distal end of the insertion section and grasp a tissue;
a cutting member that moves along a predetermined flow line to thereby cut a lumen tissue held between the pair of jaws;
a pair of sealing portions that are provided on both sides of the flow line so as to sandwich the flow line and extend parallel to the flow line; and
a tissue sealing portion that is provided on an extension line of the flow line ahead of the flow line.

9. The tissue ablation apparatus according to Claim 8,
wherein the tissue sealing portion is held between the pair of grasping members, and joins the tissue located closer to the distal end side than the flow line.

10. The tissue ablation apparatus according to Claim 8,
wherein the pair of sealing portions extend toward the distal end side than the flow line, and
wherein the tissue sealing portion is provided between the pair of sealing portions.

11. The tissue ablation apparatus according to Claim 8,
wherein the pair of sealing portions are a pair of staple rows configured by a plurality of staples arranged on both sides of the flow line.

12. The tissue ablation apparatus according to Claim 8,
wherein the tissue sealing portion is a sealing staple arranged on the distal end side of the flow line.

13. The tissue ablation apparatus according to Claim 8,
wherein the tissue sealing portion is a pair of electrodes that are held between the pair of grasping members and are capable of coming into contact with the tissue located closer to the distal end side than the flow line.

14. The tissue ablation apparatus according to Claim 8,
wherein the tissue sealing portion is a pair of heat generating elements that are held between the pair of grasping members and are capable of coming into contact with the tissue located closer to the distal end side than the flow line.
